# EUROPÄISCHE PATENTANMELDUNG

(11) **EP 0 844 231 A1**
(43) Veröffentlichungstag der Anmeldung: **27.05.1998**
(21) Anmeldenummer: 97119765.2
(22) Anmeldetag: 12.11.1997
(51) Int. Cl.: C07C 67/303, C07C 69/40

(54) **Verfahren zur Herstellung von Bernsteinsäuredialkylestern**

(30) Priorität: 25.11.1996 DE 19648761
(71) Anmelder: BAYER AG, 51368 Leverkusen (DE)
(72) Erfinder: Darsow, Gerhard, Dr., 47804 Krefeld (DE)

(57) **Zusammenfassung**

Maleinsäure-dialkylester können durch eine katalytische Flüssigphasenhydrierung mit Wasserstoff zu Bernsteinsäure-dialkylestern hydriert werden, wobei man die Hydrierung kontinuierlich bei einem Druck von 50 bis 400 bar und einer Reaktionstemperatur von 30 bis 160°C an im Festbett angeordneten sauerstofffreien und trägerfreien Formkörpern aus verpreßten Pulvern der Elemente der Eisenuntergruppe der VIII. Nebengruppe der Periodensystems der Elemente (Mendelejew) bzw. ihrer Legierungen oder Gemische untereinander mit Zusätzen von Elementen der IV. und/oder V. Nebengruppe durchführt; daneben können hydrierinerte Elemente vorliegen. Die Formkörper besitzen eine Druckfestigkeit von 20 bis 220 N und eine innere Oberfläche von 10 bis 100 m²/g.

## Beschreibung

Die vorliegende Erfindung betrifft ein kostengünstiges, kontinuierlich arbeitendes Verfahren zur Herstellung von Bernsteinsäure-dialkylestern in besonders hohen Ausbeuten aus Maleinsäure-dialkylestern, bei welchem nur sehr geringe Mengen des üblicherweise bei der Hydrierung von Maleinsäureestern entstehenden Butandiols-1,4 sowie keine Mono- und keine Hydroxycarbonsäuren der C-Zahlen <4 gebildet werden.

Bernsteinsäure-dialkylester sind wichtige, biologisch gut abbaubare Lösungsmittel für Lacke und Weichmacher für thermoplastische Polyester mit besonderen mechanischen und chemischen Eigenschaften.

Es ist bekannt, Bernsteinsäure-dialkylester durch Veresterung von Bernsteinsäure oder Bernsteinsäureanhydrid mit den entsprechenden Monoalkoholen herzustellen, wobei häufige acide Komponenten als Veresterungskatalysatoren zum Einsatz kommen und mit hohen Alkoholüberschüssen gearbeitet wird. Es ist ferner bekannt, Maleinsäure-dialkylester mit Wasserstoff in einem Suspensionsverfahren mit einem pulverförmigen Pd/Al₂O₃-Katalysator diskontinuierlich zu den korrespondierenden Bernsteinsäure-dialkylestern zu hydrieren (EP 190 424). Der Reaktionsverlauf läßt sich durch das folgende Reaktionsschema veranschaulichen:

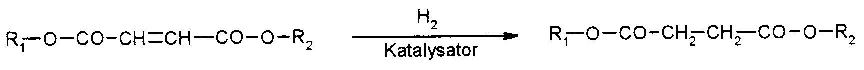

Hierbei können R₁ und R₂ gleiche oder unterschiedliche n- oder iso-Alkylreste der C-Zahlen 1 bis 12 oder cyclische Alkylreste der C-Zahlen 3 bis 6 sein.

Diskontinuierliche Verfahren haben den Nachteil, daß ihre Kapazität relativ zum Reäktionsvolumen sehr klein ist und somit ein Bedarf nach großen Reaktionsapparaturen und Lagertanks besteht. Energieverbrauch und Personalbedarf sind verhältnismäßig hoch. Kontinuierliche Pulverkatalysatorverfahren, die mit mehreren in Kaskade geschalteten Hydrierreaktoren arbeiten, vermeiden einen Teil dieser Nachteile.

Es bleibt jedoch das Erfordernis, die pulverförmigen Katalysatoren mehrfach gezielt zu dosieren, umzupumpen und quantitativ vom Reaktionsprodukt abzufiltrieren Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern, so daß mit hohen Katalysatorverbräuchen gerechnet werden muß. Es ist daher vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind. Außerdem ist man immer bestrebt, besonders hohe Katalysatorumsätze (g Maleinsäure-dialkylester / l Katalysator * h) zu erzielen.

Überraschenderweise wurde nun gefünden, daß man Maleinsäure-dialkylester in besonders hohen Ausbeuten kontinuierlich über im Festbett angeordneten trägerfreien Formkörpern aus sauerstofffreien Metallpulvern von einem oder mehreren Elementen der Eisenuntergruppe der VIII. Nebengruppe des Periodensystems (Mendelejew), legiert mit einem oder mehreren Elementen der IV. und/oder V. Nebengruppe des Periodensystems, zu den korrespondierenden Bernsteinsäure-dialkylestern hydrieren kann. Dabei können die zum Einsatz kommenden Pulver zusätzlich Anteile nicht katalytisch wirkender Elemente (z.B. Silicium, Aluminium, Kohlenstoff) enthalten, ohne daß die hohe Aktivität gemindert wird. Die Festkörper müssen eine Druckfestigkeit von 20 bis 220 N und eine innere Oberfläche von 10 bis 100 m²/g aufweisen.

Zum Einsatz kommen vorzugsweise Maleinsäure-dialkylester mit einer Reinheit ≥ 99 %. Es lassen sich aber auch Maleinsäure-dialkylester mit geringerer Reinheit nahezu quantitativ umsetzen.

Gegenstand der Erfindung ist damit ein Verfahren zur kontinuierlichen Herstellung von Bernsteinsäure-dialkylestern der Formel

R¹-O-CO-CHR³-CHR⁴-CO-O-R² (I),

in der
- R¹ und R²: unabhängig voneinander geradkettiges oder verzweigtes C₁-C₁₂-Alkyl oder C₃-C₆-Cycloalkyl bedeuten,
- R³: für Wasserstoff, Chlor oder geradkettiges oder verzweigtes C₁-C₁₂-Alkyl steht und
- R⁴: Wasserstoff oder geradkettiges oder verzweigtes C₁-C₄-Alkyl bedeutet,
durch katalytische Hydrierung von Maleinsäure- oder Fumarsäure-dialkylestern der Formel

R¹-O-CO-CR³ = CR⁴-CO-O-R² (II),

in der R¹, R², R³ und R⁴ die genannte Bedeutung besitzen,
das dadurch gekennzeichnet ist, daß die Hydrierung in der flüssigen Phase bei einem H₂-Druck von 50 bis 400 bar mit einer 20- bis 60-fachen molaren H₂-Menge, bezogen auf die stöchiometrische Menge, und bei einer Temperatur von 30 bis 160°C an im Festbett angeordneten sauerstofffreien und trägerfreien Katalysatoren durchgeführt wird, die als verpreßte, aus Metallpulvern hergestellte Formkörper vorliegen, die eine Druckfestigkeit von 20 bis 220 N und eine innere Oberfläche von 10 bis 100 m²/g aufweisen und bei denen die Metallpulver mindestens 50 Gew.-% eines oder mehrerer Elemente der Eisengruppe des Periodensystems der Elemente (Mendelejew), legiert mit mindestens 6 Gew.-% eines oder mehrerer Elemente der IV. und/oder V. Nebengruppe und 0 bis 20 Gew.-% eines oder mehrerer hydrierinerter Elemente aus der Gruppe von Aluminium, Silicium und Kohlenstoff, alles bezogen auf das Gesamtgewicht des Metallpulvers, enthalten.

Die Druckfestigkeit der trägerfreien Formkörper kann nach DIN 50106 bestimmt werden. Die Überprüfung von trägerfreien Formkörpern auf die anspruchsgemäßen inneren Oberflächen und damit auf Brauchbarkeit für das erfindungsgemäße Verfahren kann nach Methoden durchgeführt werden, die von F.M. Nelsen und F.T. Eggertsen, Analyt. Chem. 30 (1958), S. 1387 - 1390 bzw. S.J. Gregg und K.S.W. Sing, Adsorption, Surface Area and Porosity, London 1982, Kap. 2 und 6, beschrieben worden sind.

Die Eisengruppe der VIII. Nebengruppe des Periodensystems (Mendelejew) enthält die Elemente Eisen, Kobalt und Nickel. Die erfindungsgemäß zu verwendenden trägerfreien Formkörper enthalten eines oder mehrere dieser Elemente in Mengen von mindestens 50, vorzugsweise mindestens 60, insbesondere mindestens 65 Gew.-%, bezogen auf das Gesamtgewicht der trägerfreien Formkörper.

Die IV. Nebengruppe des Periodensystems enthält die Elemente Titanium, Zirkonium und Hafnium. Die V. Nebengruppe des Periodensystems enthält die Elemente Vanadium, Niob und Tantal. Die erfindungsgemäß zu verwendenden trägerfreien Formkörper enthalten eines oder mehrere dieser Elemente in Mengen von mindestens 6,0, vorzugsweise mindestens 7,5, insbesondere mindestens 9,0 Gew.-%, bezogen auf trägerfreie Formkörper; sie enthalten eines oder mehrere dieser Elemente in Mengen von höchstens 30, vorzugsweise höchstens 20 und insbesondere höchstens 15 Gew.-%, bezogen auf das Gesamtgewicht der trägerfreien Formkörper.

Die erfindungsgemäß zu verwendenden trägerfreien Formkörper können darüberhinaus, jeweils bezogen auf das Gesamtgewicht der trägerfreien Formkörper, bis zu 20, vorzugsweise bis zu 15 Gew.-% andere Elemente enthalten; Beispiele solcher Elemente, die nicht katalytisch wirken, umfassen Aluminium, Silicium und Kohlenstoff. Nach einer bevorzugten Ausführungsform enthalten die trägerfreien Formkörper außer den Metallen der XIII. und IV. und/oder V. Nebengruppe nicht mehr als 15 Gew.-% Aluminium und nicht mehr als 5 Gew.-% andere der genannten hydrierinerten Elemente.

Die Herstellung der trägerfreien Formkörper kann nach gebräuchlichen Methoden durch Verpressen der Metallpulver auf Tablettier- und Pelletiermaschinen unter hohem Druck erfolgen, wobei zur Verbesserung des Haftvermögens der Metallpartikel auch Graphit in Mengen von 0,5 bis 1,5 Gew.-%, bezogen auf das Gesamtgewicht der den Katalysator bildenden Bestandteile, oder Klebestoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden Am wirksamsten und für die Reaktionsführung am günstigsten sind tablettierte und pelletierte Formkörper in Form von Kugeln oder Zylindern mit Durchmessern von 3 bis 7 mm. Von erheblicher Bedeutung ist die Druckfestigkeit der Formkörper, die erfindungsgemäß bei Werten von 20 bis 220 N, bevorzugt 70 bis 140 N, liegt. Niedrigere Druckfestigkeiten führen zu Formkörperzerfall bzw. erosivem Abrieb, was eine metallische Kontaminierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 100 m²/g liegt und ausschlaggebend für einen möglichst quantitativen Umsatz der Einsatzstoffe ist. Die Formkörper haben makroskopisch eine glatte Oberfläche.

Für den Hydrierprozeß wird vorkomprimierter reiner Wasserstoff mit einem Druck von 50 bis 400 bar, bevorzugt 100 bis 300 bar, eingesetzt, wobei man mit einer 20-bis 60-fachen, bevorzugt 20- bis 40-fachen molaren Wasserstoffmenge, bezogen auf die stöchiometrische Menge, arbeitet.

Der Hydrierprozeß wird bei Temperaturen von 30 bis 160°C, vorzugsweise 40 bis 100°C, durchgeführt. Niedrige Temperaturen bedingen höhere Verweilzeiten oder den Verzicht auf einen quantitativen Umsatz. Höhere Temperaturen führen zur Bildung von Butandiol-1,4 und Esteralkohol als Nebenprodukte.

Die stündliche Katalysatorbelastung kann 600 bis 1.500 g Maleinsäure-dialkylester/l Katalysator betragen.

Die Hydrierung erfolgt kontinuierlich im Festbettverfahren an den als Hydrierungskatalysatoren dienenden trägerfreien Formkörpern der beschriebenen Art, indem man die zu hydrierenden flüssigen Maleinsäure-dialkylester entweder im Gleichstrom mit dem zuvor zugemischten Wasserstoff von unten nach oben aufsteigend über die in den Hydrierreaktor gefüllten Formkörper strömen läßt oder auch dem von oben einströmenden Wasserstoff von unten kommend entgegengeführt (Gegenstromverfahren).

Das erfindungsgemäße Verfahren kann natürlich auch in Lösungsmitteln durchgeführt werden. Geeignete, unter den Reaktionsbedingungen inerte Lösungsmittel sind beispielsweise Di-n-propyl-ether, Di-iso-propyl-ether, Di-n-butyl-ether, Tetrahydrofuran, Dioxan, γ-Butyrolacton. Es kann auch in Gegenwart des zu bildenden Bernsteinsäure-dialkylesters gearbeitet werden.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anordnung auf Horden (Drahtkörbe o.ä.) nützlich sein kann, oder aber ein ummanteltes Hochdruckrohrbündel, dessen Eirzelrohre mit Formkörpern ganz oder teilweise gefüllt werden.

Unter den geschilderten Reaktionsbedingungen sind auf diese Weise ganz unerwartet hohe Katalysatorstandzeiten von 15.000 Stunden und mehr zu erzielen, was zu Katalysatorverbräuchen < 0,05Gew.-%, bezogen auf hergestelltes Reaktionsprodukt, führt; so niedrige Katalysatorverbräuche konnten bisher bei der Hydrierung von Maleinsäureestern noch nicht erreicht werden.

Das den Hydrierreaktor verlassende Reaktionsgemisch wird entspannt, wobei man den überschüssigen Wasserstoff abfangen und nach erfolgtem Komprimieren und Ergänzung von verbrauchtem Wasserstoff erneut zum Einsatz bringen kann. Bei einem 99,9 bis 100%igen Umsatz besteht das Reaktionsgemisch zu mindestens 90 Gew.-% aus Bernsteinsäure-dialkylestern.

Die erfindungsgemäß einzusetzenden sauerstofffreien und trägerfreien Festbettkatalysatoren neigen im Gegensatz zu trägerhaltigen Katalysatoren nicht zum 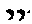 Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat entfernt werden können. Die einzusetzenden Katalysatormetalle können, etwa nach längerem Gebrauch des Katalysators leicht aufgearbeitet und wiederverwendet werden, da die Schwermetalle nicht umständlich von einem Trägermaterial getrennt werden müssen.

Erfindungsgemäß einzusetzende Ausgangsstoffe sind Maleinsäure- oder Fumarsäure-dialkylester der obigen formel (II). Geradkettiges oder verzweigtes C₁-C₁₂-Alkyl ist hierbei Methyl, Ethyl, Propyl, i-Propyl, Butyl, i-Butyl, Pentyl, i-Pentyl, Hexyl, i-Hexyl sowie die folgenden n- und i-Homologen mit 7, 8, 9, 10, 11 oder 12 C-Atomen. C₃-C₆-Cycloalkyl ist hierbei Cyclopropyl, Methyl-cyclopropyl, Dimethyl-cyclopropyl, Cyclobutyl, Cyclopentyl, Methyl-cyclopentyl oder Cyclohexyl.

In bevorzugter Weise tritt an die Stelle von R³ der Rest R¹³ mit der Bedeutung von Wasserstoff oder geradkettigem oder verzweigtem C₁-C₁₂-Alkyl, in besonders bevorzugter Weise der Rest R²³ mit der Bedeutung von Wasserstoff oder Methyl.

In weiterhin bevorzugter Weise tritt an die Stelle von R⁴ der Rest R¹⁴ mit der Bedeutung von Wasserstoff oder Methyl.

In weiterhin bevorzugter Weise sind die Ausgangsstoffe der Formel (II) Maleinsäure-dialkylester mit den Resten R¹, R², R³ und R⁴ im angegebenen Bedeutungsumfang.

In weiterhin bevorzugter Weise treten an die Stelle von R¹ und R² die Reste R¹¹ und R¹², die unabhängig voneinander geradkettiges oder verzweigtes C₁-C₆-Alkyl, Cyclopropyl oder Cyclohexyl bedeuten. In weiterhin bevorzugter Weise sind die Ausgangsstoffe der Formel (I) an der C-C-Doppelbindung nicht substituierte Maleinsäure-dialkylester mit den Resten R¹ und R² im angegebenen Bedeutungsumfang.

Die erzeugten Bernsteinsäure-dialkylester werden nach der destillativen Entfernung eines geringfügigen Leichtsiedervorlaufs und gegebenenfalls Nachlaufs in einer Reinheit von ≥ 99,9 Gew.-% erhalten und sind in dieser Qualität für alle weiterverarbeitenden Prozesse einsetzbar.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 l eines Hydrierkatalysators gefüllt, der durch Tablettierung eines Metallpulvers aus einer Ni/Zr-Legierung mit einem Zr-Gehalt von 14,9 Gew.-% und einem zusätzlichen Al-Anteil von 10,5 Gew.-% hergestellt worden war und der bei einer Zylinderhöhe von 5 mm und einem Durchmesser von 5 mm eine Druckfestigkeit von 75 N auf die Zylindermantelfläche und eine innere Oberfläche von 81 m²/g aufwies. Durch dieses Rohr wurden stündlich 1.000 g Maleinsäure-dimethylester (Reinheit ≥ 99,5 Gew.-%) gemeinsam mit der 20-fachen molaren Menge von unter einem Druck von 300 bar stehendem hochreinem Wasserstoff von unten nach oben aufsteigend gepumpt. Maleinsäure-dimethylester und Wasserstoff wurden vorab gemeinsam durch einen Wärmeaustauscher geführt und so erhitzt, daß sie in das Hochdruckrohr mit einer Temperatur von 60°C eintraten. Das das Hochdruckrohr verlassende Gemisch aus flüssigem Reaktionsprodukt und überschüssigem Wasserstoff wurde in einen Abscheider geführt, von wo der Wasserstoff nach Ersatz der verbrauchten Menge wieder zusammen mit neuem Maleinsäure-dimethylester in den Vorwärmer und von dort erneut in das Hochdruckrohr gepumpt wurde. Die farblose und klare Flüssigkeit des Reaktionsproduktes wurde nach Entspannung auf Normaldruck und Abkühlung gaschromatographisch untersucht. Sie enthielt an alkoholischen Nebenbestandteilen 0,2 Gew.-% Methanol und 0,1 Gew.-% Butandiol-1,4. Der Maleinsäure-dimethylestergehalt des organischen Reaktionsproduktes betrug < 0,1 Gew.-%, so daß der Bernsteinsäure-dimethylestergehalt bei > 99,5 Gew.-% lag. Der erzeugte Bernsteinsäure-dimethylester wurde nach der destillativen Entfernung der Nebenbestandteile in einer Reinheit von 99,9 Gew.-% erhalten und besaß einen Siedepunkt von 196°C. Der Katalysator war nach einer Laufzeit von 1.620 Stunden unverändert wirksam.

### Beispiel 2

In einem Hochdruckrohr wie in Beispiel 1 wurde bei einer Temperatur von 45°C und einem Wasserstoffdruck von 200 bar der Wasserstoff im umgekehrten Reaktionsfluß wie in Beispiel 1 dem aufsteigenden Maleinsäure-dimethylester entgegengeführt, wobei stündlich eine gleich große Menge wie in Beispiel 1 hydriert wurde. Der Katalysator war durch Tablettierung einer pulverisierten Ni-/Fe-/Zr-Legierung hergestellt worden. Die Legierung enthielt einen Eisenanteil in Nickel von 5 Gew.-% sowie einen Zr-Anteil von 10,9 Gew.-%. Die Tabletten hatten bei einer Zylinderhöhe von 5 mm und einem Durchmesser von 5 mm eine Druckfestigkeit von 107 N auf die Zylindermantelfläche und eine innere Oberfläche von 93 m²/g. Nach einer Laufzeit von 2.100 Stunden lag der Umsatz des eingesetzen Maleinsäure-dimethylesters bei 99,95 Gew.-%. Der Gehalt an Methanol im Reaktionsprodukt lag bei 0,2 Gew.-% und der Butandiol-1,4-Gehalt bei 0,1 Gew.-%, so daß der Bernsteinsäure-dimethylestergehalt des Reäktionsproduktes bei > 99,6 Gew.-% lag. Nach der destillativen Entfernung der Verunreinigungen wurde der erzeugte Bernsteinsäure-dimethylester in einer Reinheit von 99,9 Gew.-% erhalten.

### Beispiel 3

Ein senkrecht stehendes, wärmeisoliertes Hochdruckrohr aus nichtrostendem Stahl von 45 mm Innendurchmesser und 1 m Länge wurde mit 1,4 l eines Hydrierkatalysators gefüllt, der durch Tablettierung von Pulver einer Ni-/Zr-/V-Legierung mit einem Zr-Gehalt von 10,9 Gew.-%, einem V-Gehalt von 9,2 Gew.-% und einem zusätzlichen Al-Gehalt von 10,1 Gew.-% hergestellt worden war und der bei einer Zylinderhöhe von 5 mm und einem Durchmesser von 5 mm eine Druckfestigkeit von 109 N auf die Zylindermantelfläche und eine innere Oberfläche von 81 m²/g aufwies. Durch dieses Rohr wurden stündlich 1.200 g Maleinsäure-diethylester gemeinsam mit der 30-fachen molaren Menge von unter einem Druck von 300 bar stehendem hochreinen Wasserstoff von unten nach oben aufsteigend gepumpt. Maleinsäure-diethylester und Wasserstoff wurden vor Eintritt in das Hochdruckrohr auf eine Temperatur von 60°C gebracht. Nach einer Laufzeit von 1.120 Stunden lag der Umsatz des eingesetzten Maleinsäure-diethylesters bei 100 Gew.-%. Der Gehalt an Ethanol im Reaktionsprodukt lag bei 0,4 Gew.-% und der Gehalt an Butandiol-1,4 bei 0,1 Gew.-%, so daß der Bernsteinsäure-diethylestergehalt bei 99,5 Gew.-% lag. Nach der destillativen Entfernung der Verunreinigungen wurde der gewonnene Bernsteinsäure-diethylester in einer Reinheit von 99,9 Gew.-% erhalten. Er besaß einen Siedepunkt von 217°C.

### Beispiel 4

In einem Hochdruckrohr wie in Beispiel 3 wurde bei einer Temperatur von 85°C und einem Wasserstoffdruck von 200 bar stündlich eine gleich große Menge Maleinsäure-diethylester hydriert. Der Katalysator wurde durch Tablettierung einer pulverisierten Ni-/Zr-/Al-Legierung hergestellt. Die Legierung enthielt einen Zr-Anteil in Ni von 14,9 Gew.-% sowie einen Al-Gehalt von 10,5 Gew.-%. Die Tabletten hatten bei einer Zylinderhöhe von 5 mm und einem Durchmesser von 5 mm eine Druckfestigkeit von 75 N auf die Zylindermantelfläche und eine innere Oberfläche von 81 m²/g. Nach einer Laufzeit von 1.200 Stunden lag der Umsatz des eingesetzten Maleinsäure-diethylesters bei 99,9 Gew.-%. Der Gehalt an Ethanol im Reaktionsprodukt betrug 0,3 Gew.-% und der Gehalt an Butandiol-1,4 0,1 Gew.-%, so daß der Bernsteinsäure-diethylestergehalt des Reaktionsproduktes bei 99,5 Gew.-% lag.

### Beispiel 5

In einem Hochdruck wie in Beispiel 1, jedoch aus Hochdruckstahl N 9, wurden bei einer Temperatur von 90°C und einem Wasserstoffdruck von 300 bar stündlich eine Menge von 1.200 g Maleinsäure-di-n-butylester hydriert. Der Katalysator wurde durch Tablettierung von Pulver einer Ni-/Zr-/Al-Legierung mit einem Zr-Gehalt von 14,9 Gew.-% und einem Al-Gehalt von 10,5 Gew.-% hergestellt. Die Tabletten hatten bei einer Zylinderhöhe von 5 mm und einem Durchmesser von 5 mm eine Druckfestigkeit von 75 N und eine innere Oberfläche von 81 m²/g. Nach einer Laufzeit von 2.100 Stunden lag der Gehalt an Bernstein-di-n-butylester im Reaktionsprodukt bei 99,5 Gew.-%. Nach der destillativen Entfernung der Verunreinigungen wurde der erzeugte Bernsteinsäure-di-n-butylester in einer Reinheit von 99,9 Gew.-% erhalten (Kp₄: 108°C).

### Beispiel 6

In einem Hochdruckrohr wie in Beispiel 1 wurde bei einer Temperatur von 65°C und einem Wasserstoffdruck von 200 bar stündlich eine Menge von 850 g Maleinsäure-di-n-propylester hydriert. Der Katalysator wurde durch Tablettierung einer pulverisierten Ni-/V-/Si-/Al-Legierung mit einem V-Gehalt von 9,2 Gew.-%, einem Si-Gehalt von 2,8 Gew.-% und einem Al-Gehalt von 9,8 Gew.-% gewonnen. Die Tabletten hatten bei einer Zylinderhöhe von 5 mm und einem Durchmesser von 5 mm eine Druckfestigkeit von 104 N und eine innere Oberfläche von 95 m²/g. Nach einer Laufzeit von 1.100 Stunden lag der Umsatz des eingesetzten Maleinsäure-di-n-propylesters bei ≥ 99,5 Gew.-%. Der Gehalt an n-Propanol im Reaktionsprodukt betrug 0,3 Gew.-% und der Gehalt an Butandiol-1,4 0,18 Gew.-%, so daß der Bernsteinsäure-di-n-propylestergehalt bei ≥ 99,5 Gew.-% lag. Nach der Destillation des rohen Bernsteinsäure-di-n-propylesters hat dieser Siedepunkt von 248°C.

## Patentansprüche

1. Verfahren zur kontinuierlichen Herstellung von Bernsteinsäure-dialkylestern der Formel
R¹-O-CO-CHR³-CHR⁴-CO-O-R² (I),
in der
R¹ und R² unabhängig voneinander geradkettiges oder verzweigtes C₁-C₁₂-Alkyl oder C₃-C₆-Cycloalkyl bedeuten,
R³ für Wasserstoff Chlor oder geradkettiges oder verzweigtes C₁-C₁₂-Alkyl steht und
R⁴ Wasserstoff oder geradkettiges oder verzweigtes C₁-C₄-Alkyl bedeutet,
durch katalytische Hydrierung von Maleinsäure- oder Fumarsäure-dialkylestern der Formel
R¹-O-CO-CR³ = CR⁴-CO-O-R² (II),
in der R¹, R², R³ und R⁴ die genannte Bedeutung besitzen,
dadurch gekennzeichnet, daß die Hydrierung in der flüssigen Phase bei einem H₂-Druck von 50 bis 400 bar, mit einer 20- bis 60-fachen molaren H₂-Menge, bezogen auf die stöchiometrische Menge, und bei einer Temperatur von 30 bis 160°C an im Festbett angeordneten sauerstofffreien und trägerfreien Katalysatoren durchgeführt wird, die als verpreßte, aus Metallpulvern hergestellte Formkörper vorliegen, die eine Druckfestigkeit von 20 bis 220 N und eine innere Oberfläche von 10 bis 100 m²/g aufweisen und bei denen die Metallpulver mindestens 50 Gew.-% eines oder mehrerer Elemente der Eisengruppe des Periodensystems der Elemente (Mendeljew), legiert mit mindestens 6,0 Gew.-% eines oder mehrerer Elemente der IV. und/oder V. Nebengruppe und 0 bis 20 Gew.-% eines oder mehrerer hydrierinerter Elemente aus der Gruppe von Aluminium, Silicium und Kohlenstoff alles bezogen auf das Gesamtgewicht des Metallpulvers, enthalten.

2. Verfahren nach Anspruch 1, dadurch gekennzeichnet, daß die Metallpulver mindestens 60 Gew.-%, insbesondere mindestens 65 Gew.-% eines oder mehrerer Elemente der Eisengruppe enthalten.

3. Verfahren nach Anspruch 1, dadurch gekennzeichnet, daß die Metallpulver einen Gehalt von mindestens 7,5 Gew.-%, insbesondere mindestens 9 Gew.-% an Elementen der IV. und/oder V. Nebengruppe enthalten und daß sie höchstens 30, vorzugsweise höchstens 20 und insbesondere höchstens 15 Gew.-% an Elementen der IV. und/oder V. Nebengruppe enthalten.

4. Verfahren nach Anspruch 1, dadurch gekennzeichnet, daß die Metallpulver beim Vorliegen von hydrierinerten Elementen einen Gehalt von 0 bis 15 Gew.-% an Aluminium und von 0 bis 5 Gew.-% anderer hydrierinerter Elemente enthalten.

5. Verfahren nach Anspruch 1, dadurch gekennzeichnet, daß die Formkörper eine Druckfestigkeit von 70 bis 140 N aufweisen.

6. Verfahren nach Anspruch 1, dadurch gekennzeichnet, daß die Formkörper zylinder- oder kugelförmig sind und Durchmesser von 3 bis 7 mm besitzen.

7. Verfahren nach Anspruch 1, dadurch gekennzeichnet, daß die Hydrierung bei einem H₂-Druck von 100 bis 300 bar durchgeführt wird.

8. Verfahren nach Anspruch 1, dadurch gekennzeichnet, daß mit einer 20-bis 40-fachen molaren H₂-Menge gearbeitet wird.

9. Verfahren nach Anspruch 1, dadurch gekennzeichnet, daß der zu hydrierende Maleinsäure-dialkylester den Hydrierreaktor von unten nach oben aufsteigend passiert, während der für die Hydrierung benötigte Wasserstoff entweder gemeinsam mit dem Ester in den Reaktor gepumpt wird oder diesem, von oben nach unten strömend, entgegengeführt wird.
